**Europäisches Patentamt**

**European Patent Office**

**Office européen des brevets**

(19)

(11) Veröffentlichungsnummer: **0 201 866**

**A2**

(12) # EUROPÄISCHE PATENTANMELDUNG

(21) Anmeldenummer: **86106265.1**

(22) Anmeldetag: **07.05.86**

(51) Int. Cl.⁴: **G 01 N 33/49**
**G 01 N 1/28**

(30) Priorität: **10.05.85 DE 3516988**

(43) Veröffentlichungstag der Anmeldung:
**20.11.86 Patentblatt 86/47**

(84) Benannte Vertragsstaaten:
**AT BE CH DE FR GB IT LI LU NL SE**

(71) Anmelder: **Auras-Blank, Hannelore**
**Schützenstrasse 64**
**D-5810 Witten(DE)**

(72) Erfinder: **Auras-Blank, Hannelore .**
**Schützenstrasse 64**
**D-5810 Witten(DE)**

(74) Vertreter: **Kinzebach, Werner, Dr. et al,**
**Patentanwälte Reitstötter, Kinzebach & Partner**
**Sternwartstrasse 4 Postfach 86 06 49**
**D-8000 München 86(DE)**

(54) **Verfahren zum Erkennen von malignen Neoplasien.**

(57) Es wird ein Verfahren zum Erkennen von malignen Neoplasien in einer Blutprobe eines Menschen oder eines Säugetieres beschrieben, das im wesentlichen darin besteht, daß ein Blutausstrich angefertigt wird, der wesentlich dicker ist als üblich und der durch 10 bis 30-minütiges Trocknen an der Luft in eine bestimmte Konsistenz überführt wird, die den durch maligne Neoplasien bedingten Zerfallsgrad des Blutes erkennen läßt.

EP 0 201 866 A2

Croydon Printing Company Ltd.

1

Die Erfindung betrifft ein Verfahren zum Erkennen von malignen Neoplasien in einer Blutprobe eines Menschen oder eines Säugetieres.

Es gibt zahlreiche Methoden zur Diagnose und Früherkennung von Krebs beim Menschen. Diese Methoden sind jedoch entweder sehr aufwendig oder unzuverlässig oder mit unerwünschten Nebenwirkungen oder Schädigungen verbunden. Am weitesten verbreitet dürfte heute die Röntgendiagnostik sein. Mit ihr lassen sich jedoch bei weitem nicht alle malignen Neoplasien erkennen, außerdem ist an dieser Methode nachteilig, daß der Patient einer nicht unerheblichen Dosis Röntgenstrahlung ausgesetzt wird und in jedem Falle ein teures Spezialgerät erforderlich ist. Radiodiagnostische Methoden und Stoffwechseluntersuchungen sind in vieler Hinsicht noch aufwendiger und belasten den Patienten stark.

Der Erfindung liegt daher die Aufgabe zugrunde, ein Verfahren bereitzustellen, welches die Erkennung von malignen Neoplasien bei Menschen und Säugetieren außerhalb deren Körper ermöglicht, überall ohne apparativen Aufwand und ohne Laboratoriumstechnik durchführbar ist und schließlich auch bei den verschiedensten Typen maligner Neoplasien zuverlässig funktioniert.

Gelöst wird diese Aufgabe durch ein Verfahren der eingangs skizzierten Art, das dadurch gekennzeichnet ist, daß man einige Tropfen Blut direkt auf einen ersten waagrecht gehaltenen Mikroskop-Objektträger auftropft,

mit der kurzen Kante eines zweiten, gleichartigen
Objektträgers, der gegenüber dem ersten Objektträger
geneigt ist, die Blutprobe bis zur einen kurzen Kante
des ersten Objektträgers ausstreicht, in unveränderter
Haltung den zweiten Objektträger mit seiner Ausstreichkante kontinuierlich in Richtung auf die andere kurze
Kante des ersten Objektträgers so weit zurückführt,
daß etwa 4/5tel der Oberseite des ersten Objektträgers
mit einer gleichmäßigen Blutschicht bedeckt sind, den
so erhaltenen Blutausstrich 10 bis 30 Minuten bei
Raumtemperatur an der Luft trocknen läßt und anschließend
visuell makroskopisch den Zerfallsgrad des Blutes bestimmt.

Man verwendet etwa 4 bis 8 Tropfen unmittelbar entnommenen Blutes, die einen Pool im mittleren Bereich des
ersten Objektträgers bilden. Vor diesem Pool setzt man
die kurze Kante des zweiten Objektträgers etwa parallel
zur kurzen Kante des ersten Objektträgers auf letzteren
auf, zieht die Kante durch den Pool in Richtung kurze
Kante des ersten Objektträgers bis das Blut die kurze
Kante des ersten Objektträgers erreicht hat und führt
den zweiten Objektträger mit seiner Ausstreichkante
in im wesentlichen unveränderter Stellung über die
Oberfläche des ersten Objektträgers in Richtung auf
die andere kurze Kante des ersten Objektträgers so
weit zurück, daß etwa 4/5tel der Oberfläche des ersten
Objektträgers gleichmäßig mit Blut beschichtet sind.
Die Neigung des zweiten Objektträgers über dem ersten
Objektträger beträgt dabei vorzugsweise etwa 45°. Auf
diese Weise erhält man einen Blutausstrich, der sich
wesentlich von den üblichen für mikroskopische Zwecke
angefertigten Blutausstrichen unterscheidet. Aus der angegebenen Blutmenge und der angegebenen Fläche ergibt sich eine Blutschicht, die wesentlich dicker und gleichzeitig großflächiger ist als bei den bekannten Ausstrichen.

Vorteilhafterweise wird der erfindungsgemäß angefertigte Frischblutausstrich etwa 5 bis 15 Sekunden exakt waagrecht gehalten, so daß die Gleichmäßigkeit der Blutschicht gewahrt bleibt, das heißt keine Blutansammlungen entstehen. Dann wird der Objektträger mit dem Ausstrich zum Trocknen an der Luft so auf einer im wesentlichen waagrechten Unterlage abgelegt, daß diejenige kurze Kante, die den Wendepunkt der Ausstreichbewegung bildete, auf der Kante eines anderen Objektträgers aufliegt. Durch diese Art der Ablage erhält der Objektträger mit dem Ausstrich eine sehr geringe Neigung in Richtung der zuletzt ausgeführten Ausstreichbewegung, wodurch die ursprünglich etwas größere Schichtdicke im Bereich der Kante, zu der die Blutansammlung zuerst hingezogen wurde, wieder ausgeglichen wird.

Vorzugsweise läßt man den Ausstrich etwa 15 bis 20 Minuten bei Raumtemperatur an der Luft trocknen.

Es ist vorteilhaft, eine Blutprobe zu verwenden, die aus gut mit Blut versorgten, etwas tiefer unter der Haut liegenden Gefäßen bzw. Kapillaren gewonnen worden ist. Diese Blutprobe soll auf jeden Fall auch den bzw. die ersten Blutstropfen beinhalten. Vorteilhaft gewinnt man das Blut aus einer Fingerspitze unter Verwendung einer Blutlanzette als Einstichinstrument.

Das Trocknen an der Luft bei Raumtemperatur soll ohne direkte Einwirkung einer Wärme- oder Lichtquelle erfolgen.

Der in der oben beschriebenen Weise erhaltene Ausstrich wird anschließend sofort visuell ausgewertet. Blut von Patienten mit malignen Neoplasien zeigt bestimmte Zerfallserscheinungen, die umso ausgeprägter sind, je stärker die Krebsbelastung ist. Photos von Standardpräparaten können als Beurteilungsparameter herangezogen werden.

Ausführungsbeispiel:

Mit einer Blutlanzette wird eine Fingerbeere, z.B. die des vierten linken Fingers, angestochen. Der Einstich soll die tiefer unter der Haut liegenden Gefäße bzw. Kapillaren erreichen. Die ersten 4 bis 8 Tropfen läßt man aus der Einstichstelle direkt auf einen trockenen, gereinigten, nicht mit Alkohol behandelten üblichen Objektträger für Mikroskopie so auftropfen, daß sie einen "Pool" etwa in der Mitte des Objektträgers bilden. Sodann wird die kurze Kante eines zweiten Objektträgers parallel zur kurzen Kante des ersten Objektträgers vor dem Pool auf die Oberfläche des ersten Objektträgers aufgesetzt und dann durch den Pool in Richtung auf die kurze Kante des ersten Objektträgers gezogen. Dabei ist der zweite Objektträger etwa 45° gegenüber dem ersten Objektträger geneigt. Sobald der Blutausstrich die kurze Kante des ersten Objektträgers erreicht hat, wird die Ausstrichkante des zweiten Objektträgers in unveränderter Haltung in Richtung auf die andere kurze Kante des ersten Objektträgers zurückgeführt, bis etwa 4/5tel der Fläche des ersten Objektträgers mit einer gleichmäßigen Blutschicht bedeckt sind. Bei richtiger Arbeitsweise wird ein optimaler Ausstrich mit einem einzigen Hin- und Her-Bewegungszyklus der Ausstreichkante des zweiten Objektträgers erreicht.

Je nach Konsistenz des Blutes wird der Ausstrich anschließend etwa 5 bis 15 Sekunden in exakt waagrechter
Lage gehalten und darauf geachtet, daß die Schicht gleichmäßig bleibt, das heißt daß keine Blutansammlung entsteht... Bei Blut dünner Konsistenz muß man unter Umständen etwas länger als 15 Sekunden darauf achten, daß
keine Blutansammlungen entstehen. Anschließend wird
der Objektträger mit dem Ausstrich zum Trocknen an der
Luft auf einer im wesentlichen waagrechten Unterlage so
abgelegt, daß diejenige kurze Kante, die den Wendepunkt
der Ausstreichbewegung bildete, auf der Kante eines
anderen Objektträgers aufliegt. Dadurch erhält der Objektträger mit dem Ausstrich eine sehr geringfügige
Neigung in Richtung der zuletzt ausgeführten Ausstreichbewegung. Je nach Konsistenz des Blutes läßt man etwa
10 bis 30 Minuten bei Raumtemperatur an der Luft trocknen, wobei darauf zu achten ist, daß dieses Trocknen
ohne direkte Einwirkung einer Wärme- oder Lichtquelle
erfolgt. Blut dicker Konsistenz ist schon ab etwa
15 Minuten in einem die Diagnose auswertbaren Zustand.
Blut dünner Konsistenz    sollte 20 bis 30 Minuten an
der Luft "trocknen". In Wirklichkeit ist dies kein
eigentlicher Trocknungsprozeß sondern mehr oder weniger
ein Gelier- oder Abbinde-Prozeß. Unmittelbar im Anschluß
daran erfolgt die visuell, makroskopische Auswertung,
wobei der Zerfallsgrad des Blutes ermittelt wird, der
Auskunft über das Stadium der Krebsbelastung gibt.

Die Vorteile des erfindungsgemäßen Verfahrens liegen auf
der Hand. Man benötigt weder ein Röntgengerät noch andere Laboratoriumseinrichtungen. Der Test ist in jeder
Arztpraxis ohne Einsatz von Diagnosereagenzien in
kürzester Zeit durchführbar.

## Patentansprüche

1. Verfahren zum Erkennen von malignen Neoplasien in einer Blutprobe eines Menschen oder eines Säugetieres,

dadurch gekennzeichnet , daß

man einige Tropfen Blut direkt auf einen ersten, waagrecht gehaltenen Mikroskop-Objektträger auftropft,

mit der kurzen Kante eines zweiten, gleichartigen Objektträgers, der gegenüber dem ersten Objektträger geneigt ist, die Blutprobe bis zur einen kurzen Kante des ersten Objektträgers ausstreicht, in unveränderter Haltung den zweiten Objektträger mit seiner Ausstreichkante kontinuierlich in Richtung auf die andere kurze Kante des ersten Objektträgers so weit zurückführt, daß etwa 4/5tel der Oberseite des ersten Objektträgers mit einer gleichmäßigen Blutschicht bedeckt sind,

den so erhaltenen Blutausstrich 10 bis 30 Minuten bei Raumtemperatur an der Luft trocknen läßt und

anschließend visuell makroskopisch den Zerfallsgrad des Blutes bestimmt.

M/26 082 2

2. Verfahren nach Anspruch 1, dadurch gekennzeichnet, daß man von 4 bis 8 etwa auf die Mitte des ersten Objektträgers aufgebrachten Tropfen unmittelbar entnommenen Blutes ausgeht.

3. Verfahren nach Anspruch 1, dadurch gekennzeichnet, daß die Neigung des zweiten Objektträgers gegenüber dem ersten Objektträger etwa 45° beträgt.

4. Verfahren nach einem der vorhergehenden Ansprüche, dadurch gekennzeichnet, daß man den ersten Objektträger unmittelbar nach Anfertigen des Blutausstriches etwa 5 bis 15 Sekunden waagrecht hält, so daß sich keine Blutansammlungen bilden, dann den Objektträger mit dem Ausstrich zum Trocknen an der Luft so auf einer im wesentlichen waagrechten Unterlage ablegt, daß diejenige kurze Kante, die den Wendepunkt der Ausstreichbewegung bildete, auf der Kante eines anderen Objektträgers aufliegt.

5. Verfahren nach einem der vorhergehenden Ansprüche, dadurch gekennzeichnet, daß man den Blutausstrich etwa 15 bis 20 Minuten bei Raumtemperatur an der Luft trocknen läßt.

6. Verfahren nach einem der vorhergehenden Ansprüche, dadurch gekennzeichnet, daß man eine Blutprobe verwendet, die aus gut mit Blut versorgten, etwas tiefer unter der Haut liegenden Gefäßen bzw. Kapillaren gewonnen worden ist und auch den ersten Blutstropfen beinhaltet.